# EUROPÄISCHE PATENTSCHRIFT

(11) **EP 1 038 508 B1**
(45) Veröffentlichungstag und Bekanntmachung des Hinweises auf die Patenterteilung: **20.07.2005**
(21) Anmeldenummer: 00105616.7
(22) Anmeldetag: 16.03.2000
(51) Int. Cl.: A61F 2/00

(54) **Flächiges Implantat**
Prosthetic patch
Patch prothétique

(30) Priorität: 20.03.1999 DE 19912648
(43) Veröffentlichungstag der Anmeldung: 27.09.2000
(73) Patentinhaber: Aesculap AG & Co. KG, 78532 Tuttlingen (DE)
(72) Erfinder: Goldmann, Helmut, Dr., 78532 Tuttlingen (DE)
(74) Vertreter: Patentanwälte Ruff, Wilhelm, Beier, Dauster & Partner

(56) Entgegenhaltungen:
- WO-A-89/08467
- WO-A-90/14810
- WO-A-92/10218
- WO-A-98/49967
- WO-A-99/51163
- CA-A- 2 114 282
- FR-A- 2 145 975

## Beschreibung

Die vorliegende Erfindung betrifft ein flächiges Implantat.

Der Eingeweidebruch, die sogenannte Hernie, stellt eine häufige Erkrankung dar. Hierbei handelt es sich im allgemeinen um ein Hindurchtreten von Organen oder Organteilen aus der natürlichen Körperhöhle durch eine vorgebildete oder erworbene Lücke. Unter den äußeren Brüchen, bei denen stets der Bruchsack vom Bauchfell umschlossen wird, sind Leisten-, Nabel- und Narbenbrüche die häufigsten Formen. Ursachen für das Auftreten von Hernien sind vor allem Muskel- oder Bindegewebsschwächen in Zusammenhang mit Überbelastungen, altersbedingter Erschlaffung, angeborener Schwächung der Bauchdecke oder ungenügende Narbenbildung nach einem Leibesschnitt (Narbenbruch).

Eine effektive Behandlung ist in den meisten Fällen durch einen operativen Eingriff möglich, bei dem der Bruchinhalt aus dem Bruchsack in den Bauch zurückverlagert wird und die Bruchpforte verschlossen wird. Dieser Verschluß der Bruchpforte erfolgt konventionell durch eine Naht.

Diese chirurgische Technik hat jedoch den Nachteil, daß es in bis zu 20 % der Fälle zum erneuten Bruch, dem sogenannten Bruchrezidiv kommen kann.

Wegen dieser unbefriedigenden Rezidivrate nach der konventionellen Hernienoperation werden in der modernen Hernienchirurgie zunehmend künstliche Verstärkungsmaterialien zur Bauchwandrekonstruktion verwendet. Hier spielen besonders Polypropylen- aber auch Polyesternetze eine Rolle.

Obwohl die Verwendung solcher Netze offensichtlich zu einer deutlichen Verringerung der Rezidivrate geführt hat, sind diese Implantate nicht unproblematisch wegen möglicher Infektionen oder Fistelbildungen, besonders aber wegen des Risikos von Weichteiladhäsionen.

Bei den bisherigen Implantaten handelt es sich um offene textile Strukturen, die ein Anhaften von Zellen sowie ein Durchwachsen von Zellen begünstigen. Dies ist einerseits von Vorteil, da es eine feste Verbindung des Implantats mit der Bauchdecke ausbildet und somit die gewünschte Stützfunktion gewährleistet. Andererseits kann es zu schwer lösbaren Verwachsungen im Bauchraum mit Verhärtungen (Narbenpanzer), Beeinträchtigungen des Darms sowie der inneren Organe und damit verbundenen Beschwerden beim Patienten führen.

Es sind auch flächige textile Implantate bekannt, die mit einem Imprägnierungsmittel versiegelt sind. Hier zeigen sich aber Probleme bei der Fixierung an der Bauchwand, insbesondere bei langandauerndem Verbleib im Körper.

Ein flächiges implantat zur Verwendung in der Chirurgie gemäß den Merkmalen des Oberbegriffs des Anspruchs 1 ist aus dem Dokument CA-A-2 114 282 bekannt.

Es stellt sich die Aufgabe, ein Implantat zum Einsatz bei chirurgischen Eingriffen zur Verfügung zu stellen, das die Schwierigkeiten von Implantaten aus dem Stand der Technik überwindet, das einfach und kostengünstig herzustellen und nach üblichen chirurgischen Methoden einsetzbar ist.

Diese Aufgabe wird gelöst durch ein flächiges Implantat nach Anspruch 1. Das erfindungsgemäße Implantat erlaubt eine gute Verankerung von Körperzellen auf der der Bauchwand zugewandten strukturierten Seite und verhindert ein unerwünschtes Verwachsen mit Organen und Körperteilen auf der dem Bauchinnenraum zugewandten im wesentlichen geschlossenen Seite.

Zur Verwendung in der Chirurgie stellt die Erfindung ein flächiges Implantat zur Verfügung mit einem flexiblen Flächengebilde, das auf einer Seite eine im wesentlichen geschlossene Oberfläche besitzt und auf der anderen Seite eine dreidimensionale Mikrostruktur, die ein Einwachsen von Zellen erlaubt. Die im wesentlichen geschlossene Oberfläche ist mikroporös, wobei die Mikroporen so klein sind, daß sie einen Stoffaustausch erlauben, aber das Einwachsen von Zellen im wesentlichen verhindern.

Bevorzugt kann die im wesentlichen geschlossene Oberfläche glatt ausgebildet sein. Ferner ist die im wesentlichen geschlossene Oberfläche von einer mit dem flexiblen Flächengebilde verbundenen Oberflächenschicht, insbesondere einer Beschichtung des Flächengebildes ausgebildet.

Erfindungsgemäß besitz die dreidimensionale Mikrostruktur hintergreifbare Stellen zum Einwachsen von Zellen. Mit Vorteil kann das flexible Flächengebilde von einem porösen flexiblen Strukturmaterial, insbesondere einem flexiblen Träger gebildet sein und die dreidimensionale Mikrostruktur von der freiliegenden Oberflächenstruktur des Strukturmaterials gebildet sein. Als Beispiele für poröses flexibles Strukturmaterial sind offenzelliger Strukturschaum oder eine Gitterstruktur zu nennen.

Mit Vorteil kann das Implantat aus mindestens einem synthetischen Polymermaterial gebildet sein. Bevorzugt kann das Flächengebilde des Implantats gemäß der Erfindung aus Polypropylen, Polyester, Polytetrafluorethylen und/oder Polyester und Polytetrafluorethylen gebildet sein. Gemäß der Erfindung besonders bevorzugt ist ein flächiges Implantat aus Polyester und Polytetrafluorethylen. Es können auch resorbierbare Materialien, wie Polylactide, Polyglycolide und Copolymere davon Anwendung finden, wenn eine Resorbierbarkeit bzw. Teilresorbierbarkeit erwünscht ist.

In einer Ausführungsform der Erfindung kann das flexible Strukturmaterial ein Textilmaterial, insbesondere ein poröser textiler Träger sein. Bevorzugt kann der flexible textile Träger mindestens auf der Seite mit der dreidimensionalen Mikrostruktur eine bei Gefäßimplantaten bekannte offene Textilstruktur aufweisen, die insbesondere von texturierten Garnen, Flottungen und/oder Veloursschlingen gebildet ist, wie sie beispielsweise aus den Veröffentlichungen US 4047252, US 3878565, DE 2461370 und US 4517687 bekannt sind.

Es können schrumpffähige Fasern und daraus hergestellte Garne wie andere Fasern und Garne auch nach üblichen Verfahrenstechniken zu textilen Gebilden verarbeitet werden. Anschließend werden durch eine Schrumpfbehandlung die schrumpffähigen Fasern geschrumpft, was bei daraus hergestellten Textilgebilden zu einer Verdichtung der Struktur führt. Durch den planmäßigen Einatz von schrumpffähigen Fasern und nicht schrumpffähigen Fasern in Kombination können gezielt Modifikationen der Textilstruktur vorgenommen werden.

Erfindungsgemäß kann das Flächengebilde nach einer textilen Technik, insbesondere Wirken, Stricken, Weben oder Flechten ausgebildet sein. Solche Verfahrenstechniken sind den Fachleuten bekannt, so daß auf eine detaillierte Ausführung hier verzichet wird. Auf diese Weise ist eine einfache und kostengünstige Herstellung nach bekannten und in der Praxis bewährten Verfahrenstechniken und mit üblichen Maschinen und Werkzeugen möglich.

Mit Vorteil kann das flexible textile Flächengebilde, insbesondere der textile Träger, eine Webware oder eine insbesondere gewirkte Maschenware sein, die mindestens auf der dreidimensional strukturierten Seite freiliegende, als Verankerung für Zellen dienende Faser oder Fäden aufweist.

Es können textile Fasermaterialien wie synthetische Monofilamente, multifile Fäden oder multifile Garne verwendet werden. Gemäß der Erfindung bevorzugt kann das Flächengebilde mindestens teilweise mit multifilen Garnen ausgebildet sein. Diese Garne können glatt oder texturiert sein. In einer Ausführungsform der Erfindung können die Garne aus nur einer Art von Fasermaterial gebildet sein. In einer anderen Ausführungsform können die Garne aus mehreren Fasermaterialien gebildet sein. Die Garne können mindestens zum Teil aus hoch schrumpffähigem Fasermaterial gebildet sein. Sie können auch Mischungen aus nichtresorbierbaren und resorbierbaren Fasermaterialien sein.

Das erfindungsgemäße Implantat kann sich insbesondere dadurch auszeichnen, daß eine Fläche mit einer strukturierten Oberfläche ausgebildet ist. In einer bevorzugten Ausführungsform kann die strukturierte Oberfläche als Velours ausgebildet sein. In einer besonders bevorzugten Ausführungsform der Erfindung kann das Flächengebilde als Velours, insbesondere als Einfachvelours ausgebildet sein. In Weiterbildung der besonders bevorzugten Ausführungsform kann das Flächengebilde als Doppelvelours ausgebildet sein. Insbesondere kann der Doppelvelours mit unterschiedlichen Polhöhen auf beiden Seiten des Flächengebildes ausgebildet sein.

In einer bevorzugten Ausführungsform der Erfindung kann das flexible Flächengebilde, insbesondere der texturierte Träger ein Velours, insbesondere ein Doppelvelours sein, wobei ein Doppelvelours an der strukturierten Seite vorzugsweise eine größere Polhöhe aufweist als auf der im wesentlichen geschlossenen Seite des Implantats.

Erfindungsgemäß kann sich das flächige Implantat dadurch auszeichnen, daß die der strukturierten Oberfläche gegenüberliegende Seite als glatte Oberfläche ausgebildet ist. Glatt bedeutet in diesem Fall, daß die Oberfläche keine Strukturierung, z. B. durch die Textilkonstruktion aufweist, die eine Verankerung von Zellverbänden ermöglicht. Ferner bedeutet glatt, daß keine Fasern des verwendeten Textilmaterials aus der Fläche herausragen.

In einer anderen Ausführungsform kann die im wesentlichen geschlossene Oberfläche durch Aufbringen einer Polymerfolie ausgebildet sein. eine solche Polymerfolie kann mit Vorteil als mikroporöser Film ausgebildet sein. Als geeignetes Material für eine Polymerfolie zur Modifizierung des Implantats gemäß der Erfindung kann ein für die vorgesehene Anwendung als chirurgisches Implantat geeignetes bioverträgliches Material ausgewählt sein, bevorzugt Polypropylen, Polyester, Polytetrafluorethylen und/oder Polyurethan. Das Aufbringen einer Folie aus synthetischem Material zur Modifizierung der Oberfläche des textilen Flächengebildes gemäß der Erfindung kann nach Verfahrenstechniken wie beispielsweise Aufkaschieren oder Aufkleben erfolgen.

In einer anderen Ausführungsform der Erfindung kann das Implantat von einem dreidimensionalen Vlies gebildet sein, wobei Fasermaterialien auf der Seite mit im wesentlichen geschlossener Oberfläche dicht beieinander liegen und auf der Seite mit Mikrostruktur einen offenen Verbund bilden.

Die im wesentlichen geschlossene Oberfläche und insbesondere das gesamte Implantat besitz eine Luftdurchlässigkeit von 5 bis 100 ml Luft/cm²·min, insbesondere 25 bis 75 ml Luft/cm²·min, bei einer Druckdifferenz von 1,2 kPascal. Bei einer solchen Mikroporosität ist ein Stoffaustausch im molekularen Bereich möglich. Dies begünstigt Stoffwechselvorgänge im Bereich des Implantats, fördert die Zufuhr von lebenswichtigen Nährstoffen und Aufbaustoffen sowie die Abfuhr von Stoffwechselschlacken und Schadstoffen. Auf diese Weise ist eine gute Verträglichkeit und erfolgreiches Heilen vorteilhaft möglich. Die Mikroporosität der im wesentlichen geschlossenen Oberfläche ist jedoch nicht geeignet für den Durchtritt bzw. die feste und durchgehende Verankerung von großen Partikeln wie Zellen.

Die erfindungsgemäße im wesentlichen geschlossene Oberfläche führt zu einer Verminderung der Strukturierung der Implantatoberfläche auf einer Seite. Erhebungen und Vertiefungen, die sich beispielsweise aus der Herstellungstechnik des textilen Flächengebildes ergeben, werden durch die im wesentlichen geschlossene, insbesondere glatte Oberfläche ausgeglichen. Außerdem werden einzelne Fasern, die aus dem textilen Flächengebilde hervorstehen eingeschlossen. Das Schließen der Implantatoberfläche gemäß der Erfindung kann dementsprechend als eine Art Oberflächenversiegelung betrachtet werden.

Auf diese Weise ist die im wesentlichen geschlossene Oberfläche mit ihrer geringen Porosität und geringen Oberflächenstruktur ungünstig für ein Anhaften von Zellen. Die Zellen finden keine geeigneten Verankerungsstellen, die für ihr Wachstum erforderlich sind. Auf diese Weise ist eine Besiedelung der im wesentlichen geschlossenen Implantatoberfläche mit Zellen im wesentlichen zu verhindern.

Ferner kann das erfindungsgmeäße Implantat bevorzugt ausfranssichere Ränder aufweisen, die ausfranssicher verarbeitet und/oder gebunden, insbesondere verschweißt sind.

Das Implantat hat eine Gesamtdicke von ca. 0,1 bis 1,2 mm besitzen, wobei die Dicke der im wesentlichen geschlossenen Oberfläche 3 bis 15 % davon beträgt. Außerdem kann der textile Träger eine gewebte, gewirkte oder gestrickte Grundbindung mit einer Dicke von 0,05 bis 0,4 mm besitzen und eine mindestens einseitige offene Struktur mit einer Dicke von 0,05 bis 0,8 mm.

Es ist erfindungsgemäß vorgesehen, daß alle Komponenten des Implantats bioverträglich und langzeitstabil sind. Insbesondere kann es über die gesamte Fläche teilresorbierbar sein, insbesondere die im wesentlichen geschlossene Oberfläche aus vollständig resorbierbarem Material bestehen. Auf diese Weise kommt es nach der Implantation im physiologischen Milieu des Körpers nicht zu einem Abbau der Oberfläche. Die im wesentlichen geschlossene Oberfläche des Implantats behält somit ihre zellabweisende Eigenschaft, die ein Anhaften und Einwachsen von Zellen verhindert, wie es gemäß der Erfindung vorgesehen ist.

Mit Vorteil kann das Implantat gemäß der Erfindung aus flexiblem Material sein. Bevorzugt kann die im wesentlichen geschlossene Oberfläche mit einem in einem Lösemittel löslichen unvernetzten Polymer gebildet sein. In Weiterbildung kann die im wsentlichen geschlossene Oberfläche mit einem gummielastischen Polymer gebildet sein. Erfindungsgemäß bevorzugt kann die im wesentlichen geschlossene Oberfläche in Form einer Lösung in einem niedrigsiedenden Lösemittel auf ein insbesondere textiles Flächengebilde einseitig aufgetragen sein. In einer besonders bevorzugten Ausführungsform der Erfindung kann die im wesentlichen geschlossene Oberfläche des flächigen Implantats aus unvernetztem Polyurethan gebildet sein.

In Weiterbildung kann im Implantat ein antimikrobiotischer Wirkstoff, wie beispielsweise ein Antibiotikum enthalten sein. Die Verabreichung von Antibiotika dient insbesondere der Vorbeugung von Infektionen. Zur Prophylaxe und Therapie mit Antibiotika finden im Bereich der Chirurgie beispielsweise Cefalosporine wie Cefazolin oder Cefamandol, Netilmicin, Penicilline wie Oxacillin oder Mezlocillin, Tetracyclin, Metronidazol oder Aminoglykoside wie Gentamicin oder Neomycin sowie z. B. Rifampicin Anwendung. Die Fachleute können gemäß den jeweiligen Erfordernissen einen oder mehrere geeignete Wirkstoffe zur Anwendung auswählen. Auch Wachstumsfaktoren können im Implantat enthalten sein.

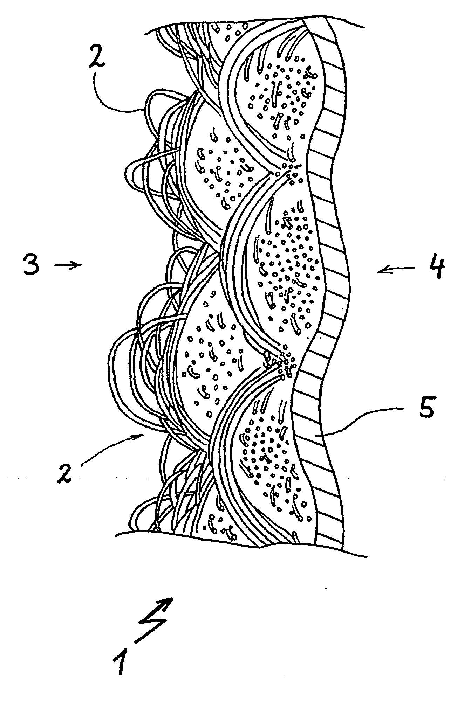

Weitere Merkmale der Erfindung ergeben sich aus der nachfolgenden Beschreibung eines bevorzugten Beispiels in Verbindung mit den Unteransprüchen und der Zeichnung. Hierbei können die einzelnen Merkmale jeweils für sich alleine oder zu mehreren bei einer Ausführungsform der Erfindung verwirklicht sein. Das Beispiel dient jedoch nur zur Erläuterung und ist nicht als Einschränkung zu verstehen.

Die Zeichnung zeigt eine schematischen Schnitt durch ein erfindungsgemäßes Implantat.

### Beispiel 1

Ein textiles Flächengebilde 1 ist als Kettengewirk aus multifilem Polyethylenterephthalatgarn in Form eines Einfachvelours ausgebildet, wobei Veloursschlingen 2 aus texturiertem Garn bestehen. Das Gewirk kann als Doppelveloursgewirk ähnlich ausgebildet sein, wie die Gewirke nach den US-Patentschriften 4047252 und 4193397. Es ist jedoch im Gegensatz zu den dort beschriebenen rohrförmigen Kettenwirkstoffen flächig ausgebildet. Es kann auch als Einfachvelours ausgebildet sein. Das Gewirk ist porös und flexibel. Es besitzt auf der Veloursseite 3 eine offene dreidimensional strukturierte Oberfläche, die aufgrund der Veloursschlingen und der Texturierung der Fasern, zahlreiche im wesentlichen gleichmäßig über die Oberfläche verteilte Hintergreifmöglichkeiten zum Einwachsen von Körperzellen aufweist. Dabei sind die Öffnungen zwischen den Garnschlingen bzw. den einzelnen Fasern groß im Verhältnis zur Größe von Körperzellen. Dies ermöglicht das Einwachsen eines zusammenhängenden Zellverbundes.

Die gegenüberliegende Seite 4 des Gewirks 1 ist aufgrund der Bindung des Gewirks dichter und eher eben. Zusätzlich weist das Gewirk 1 an dieser Seite eine Sprühbeschichtung 5 aus unvernetztem Polyurethan auf, die mit den an der Oberfläche freiliegenden Fasern des Gewirks 1 verbunden ist und die textile Struktur an dieser Oberfläche verschließt. Die Dicke der Sprühbeschichtung liegt in der Größenordnung von etwa 1/10 bis 1/20 der Gesamtdicke von textilem Flächengebilde und Versiegelungsschicht, das heißt, der Gesamtdicke des flächigen Implantats von ca. 0,8 mm.

Durch die Technik der Sprühbeschichtung, das heißt durch Aufsprühen einer Lösung des Polyurethans in Chloroform hat die Schicht 5 einen Aufbau, der mit einem dichten Vlies in etwa vergleichbar ist, mikroporös ist und aufgrund der elastomeren Eigenschaften des Polyurethans flexibel ist. Als Polyurethan eignen sich beispielsweise bekannte lineare Polyester und Polyurethane. Durch die Versiegelungsschicht 5 ist das textile Flächengebilde 1 aus dem Veloursgewirk an der von den Veloursschlingen 2 abweisenden Seite nach Art eines Tiefenfilters im wesentlichen verschlossen. Die Mikroporen, deren Größe in etwa in der Größenordnung der Größe von Körperzellen oder kleiner ist, erlauben einen Stoffaustausch von Körperflüssigkeiten zur Aufrechterhaltung des Stoffwechsels, erlauben jedoch nicht das Einwachsen von Zellen, zumindest nicht in solcher Form, daß ein Anwachsen an Körperteile im Bereich der Bauchhöhle zu befürchten ist.

Die Versiegelungsschicht 5 dringt nur unwesentlich in die textile Oberfläche des textilen Trägers 1 ein, so daß das dreidimensionale Volumen des Flächengebildes zum Einwachsen von Zellen aus der Bauchwand zur Verfügung steht, während die im wesentlichen geschlossene Versiegelungsschicht 5 ein solches Einwachsen von Zellen auf der Seite des Bauchraumes verhindert.

Die Ränder des Flächengebildes sind aufgrund der Wirkart an sich schon ausfransfest. Die Versiegelungsschicht verhindert ein Einrollen des Gewirks und vermindert zusätzlich die Neigung zur Fransenbildung. Falls gewünscht, können die Ränder zusätzlich noch durch Verschweißen der thermoplastischen Fasern ausfransfest gemacht werden. Zur Verwendung in der Chirurgie werden Stücke von etwa 8 x 2 cm bis 30 x 30 hergestellt. Je nach Bedarf können diese Stücke vor dem Implantieren noch zurechtgeschnitten werden.

Mit dem erfindungsgemäßen Implantat durchgeführte Tierversuche ergaben gute Ergebnisse. Auf der unbeschichteten, offenen Seite der Implantate erfolgte ein sehr guter Einbau in das Gewebe mit kräftiger Vaskularisation in den Implantatmaschen und völlig fehlenden Abstoßungserscheinungen. Durch die Versiegelung der bauchhöhlenseitigen Fläche zeigten sich an dieser Seite keine Verwachsungen. Auch nach längerer Verweildauer des Implantats im Bauchraum war eine freie Beweglichkeit der Bauchorgane relativ zum Implantat gewährleistet.

## Patentansprüche

1. Flächiges Implantat zur Verwendung in der Chirurgie mit einem flexiblen textilen Flächengebilde (1), das auf einer Seite (4) eine im wesentlichen geschlossene Oberfläche (5) besitzt und auf der anderen Seite (3) eine dreidimensionale Mikrostruktur (2), die ein Einwachsen von Zellen erlaubt, **dadurch gekennzeichnet, dass** die im wesentlichen geschlossene Oberfläche (4) mikroporös ist, wobei die Mikroporen so klein sind, dass sie einen Stoffaustausch erlauben, aber das Einwachsen von Zellen im wesentlichen verhinderndie im wesentlichen geschlossene Oberfläche (5) von einer mit dem flexiblen Flächengebilde (1) verbundenen Oberflächenbeschichtung gebildet ist, dass die Beschichtung (5) ein Sprühvlies aus einem Polymer ist und die im wesentlichen geschlossene Oberfläche (5) eine Luftdurchlässigkeit von 5 bis 100 ml Luft/cm² min. besitzt, bei einer Druckdifferenz von 1,2 kPascal, das Implantat eine Gesamtdicke von ca. 0,1 bis 1,2 mm besitzt, wobei die Dicke der im wesentlichen geschlossenen Oberfläche 3 bis 15 % davon beträgt, der textile Träger (1) eine gewebte, gewirkte oder gestrickte Grundbindung mit einer Dicke von 0,05 bis 0,4 mm besitzt und eine mindestens einseitige offene Struktur mit einer Dicke von 0,05 bis 0,8 mm.

2. Implantat nach Anspruch 1, **dadurch gekennzeichnet, dass** die im wesentlichen geschlossene Oberfläche (5) im wesentlichen glatt ausgebildet ist.

3. Implantat nach Anspruch 1 oder 2, **dadurch gekennzeichnet, dass** die dreidimensionale Mikrostruktur (2) hintergreifbare Stellen zum Einwachsen von Zellen besitzt.

4. Implantat nach einem der vorhergehenden Ansprüche, **dadurch gekennzeichnet, dass** das flexible textile Flächengebilde (1) von einem porösen flexiblen Strukturmaterial, insbesondere einem flexiblen Träger, gebildet ist und die dreidimensionale Mikrostruktur von der freiliegenden Oberflächenstruktur (2) des Strukturmaterials gebildet ist.

5. Implantat nach Anspruch 4, **dadurch gekennzeichnet, dass** das flexible Strukturmaterial (2) ein poröser textiler Träger ist.

6. Implantat nach Anspruch 5, **dadurch gekennzeichnet, dass** das flexible Textilmaterial (1) insbesondere der textile Träger mindestens auf der Seite mit der dreidimensionalen Mikrostruktur eine bei Gefäßimplantaten bekannte offene Textilstruktur (2) aufweist, die insbesondere von texturierten Garnen, Flottungen und/oder Velourschlingen gebildet ist.

7. Implantat nach einem der vorhergehenden Ansprüche, **dadurch gekennzeichnet, dass** das flexible Flächengebilde (1), insbesondere der textile Träger, eine Webware oder eine insbesondere gewirkte Maschenware ist, die mindestens auf der dreidimensional strukturierten Seite (3) freiliegende, als Verankerung für Zellen dienende Fasern oder Fäden (2) aufweist.

8. Implantat nach einem der vorhergehenden Ansprüche, **dadurch gekennzeichnet, dass** das flexible Flächengebilde (1), insbesondere der texturierte Träger, ein Velours (2), insbesondere ein Doppelvelours ist, wobei ein Doppelvelours an der strukturierten Seite vorzugsweise eine größere Polhöhe aufweist als auf der im wesentlichen geschlossenen Seite des Implantats.

9. Implantat nach einem der vorhergehenden Ansprüche, **dadurch gekennzeichnet, dass** die Beschichtung (5) ein Sprühvlies aus einem in einem nichtwässrigen flüssigen, vorzugsweise flüchtigen Medium löslichen bzw. dispergierbaren Polymer ist.

10. Implantat nach einem der vorhergehenden Ansprüche, **dadurch gekennzeichnet, dass** die im wesentlichen geschlossene Oberflächenschicht (5) aus Polyurethan, insbesondere unvernetztem Polyurethan besteht.

11. Implantat nach einem der vorhergehenden Ansprüche, **dadurch gekennzeichnet, dass** das gesamte Implantat eine Luftdurchlässigkeit von 5 bis 100 ml Luft/cm² min. besitzt, bei einer Druckdifferenz von 1,2 kPascal.

12. Implantat nach einem der vorhergehenden Ansprüche, **dadurch gekennzeichnet, dass** die im wesentlichen geschlossene Oberfläche (5) und insbesondere das gesamte Implantat eine Luftdurchlässigkeit von 25 bis 75 ml Luft/cm² min. besitzt, bei einer Druckdifferenz von 1,2 kPascal.

13. Implantat nach einem der vorhergehenden Ansprüche, **dadurch gekennzeichnet, dass** es ausfranssichere Ränder aufweist, die ausfranssicher verarbeitet und/oder gebunden, insbesondere verschweißt sind.

14. Implantat nach einem der Ansprüche 1 bis 9 und 11 bis 13, **dadurch gekennzeichnet, dass** es über die gesamte Fläche teilresorbierbar ist, insbesondere die im wesentlichen geschlossene Oberfläche aus vollständig resorbierbarem Material besteht.

## Claims

1. Planar implant for use in surgery, with a flexible textile fabric (1) which, on one side (4), has a substantially closed surface (5), and, on the other side (3), has a three-dimensional microstructure (2) permitting incorporation of cells, **characterized in that** the substantially closed surface (5) is microporous, the micropores being sufficiently small that they permit substance exchange but substantially prevent incorporation of cells, the substantially closed surface (5) being formed by a surface coating connected to the flexible fabric (1), the coating (5) being a sprayed web of a polymer and the substantially closed surface (5) having an air permeability of 5 to 100 ml air/cm² min, at a pressure difference of 1.2 kPascal, the implant having a total thickness of ca. 0.1 to 1.2 mm, the thickness of the substantially closed surface amounting to 3 to 15% thereof, the textile support (1) having a woven, warp-knitted or knitted basic weave with a thickness of 0.05 to 0.4 mm and an at least unilaterally open structure with a thickness of 0.05 to 0.8 mm.

2. Implant according to Claim 1, **characterized in that** the substantially closed surface (5) is substantially smooth.

3. Implant according to Claim 1 or 2, **characterized in that** the three-dimensional microstructure (2) has locations that can be engaged behind to permit incorporation of cells.

4. Implant according to one of the preceding claims, **characterized in that** the flexible textile fabric (1) is formed by a porous flexible structure material, in particular a flexible support, and the three-dimensional microstructure is formed by the exposed surface structure (2) of the structure material.

5. Implant according to Claim 4, **characterized in that** the flexible structure material (2) is a porous textile support.

6. Implant according to Claim 5, **characterized in that** the flexible textile material (1), in particular the textile support, at least on the side with the three-dimensional microstructure, has an open textile structure (2) which is known in vascular implants and which is formed in particular by textured yarns, float stitches and/or velour loops.

7. Implant according to one of the preceding claims, **characterized in that** the flexible fabric (1), in particular the textile support, is a woven fabric or an in particular warp-knitted mesh fabric which, at least on the three-dimensionally structured side (3), has exposed fibres or filaments (2) used as anchoring for cells.

8. Implant according to one of the preceding claims, **characterized in that** the flexible fabric (1), in particular the textured support, is a velour (2), in particular a double velour, and a double velour on the structured side preferably has a greater pile height than on the substantially closed side of the implant.

9. Implant according to one of the preceding claims, **characterized in that** the coating (5) is a sprayed web of a polymer which is soluble or dispersible in a non-aqueous liquid and preferably volatile medium.

10. Implant according to one of the preceding claims, **characterized in that** the substantially closed surface layer (5) is made of polyurethane, in particular uncrosslinked polyurethane.

11. Implant according to one of the preceding claims, **characterized in that** the entire implant has an air permeability of 5 to 100 ml air/cm² min, at a pressure difference of 1.2 kPascal.

12. Implant according to one of the preceding claims, **characterized in that** the substantially closed surface (5) and in particular the entire implant has an air permeability of 25 to 75 ml air/cm² min, at a pressure difference of 1.2 kPascal.

13. Implant according to one of the preceding claims, **characterized in that** it has fray-proof edges which are processed and/or bonded, in particular welded, in a fray-proof manner.

14. Implant according to one of Claims 1 to 9 and 11 to 13, **characterized in that** it is partially absorbable across the entire surface, and in particular the substantially closed surface is made of completely absorbable material.

## Revendications

1. Implant plat destiné à être utilisé en chirurgie, comprenant une structure plane flexible textile (1) qui possède, d'un côté (4), une surface (5) essentiellement fermée et de l'autre côté (3), une microstructure tridimensionnelle (2) qui permet la croissance de cellules, **caractérisé en ce que** la surface essentiellement fermée (4) est microporeuse, les micropores étant si petits qu'ils permettent un échange de substances mais empêchent substantiellement la croissance de cellules, la surface (5) essentiellement fermée étant formée par un revêtement de surface connecté à la structure plane flexible (1), **en ce que** le revêtement (5) est un toison giclé constitué d'un polymère et la surface (5) essentiellement fermée possède une perméabilité à l'air de 5 à 100 ml d'air/cm² min., pour une différence de pression de 1,2 kPascal, l'implant possède une épaisseur totale d'environ 0,1 à 1,2 mm, l'épaisseur de la surface essentiellement fermée en constituant 3 à 15%, le support textile (1) possédant une liaison de base tissée, maillée ou tricotée ayant une épaisseur de 0,05 à 0,4 mm et une structure ouverte au moins d'un côté ayant une épaisseur de 0,05 à 0,8 mm.

2. Implant selon la revendication 1, **caractérisé en ce que** la surface essentiellement fermée (5) est réalisée de manière essentiellement lisse.

3. Implant selon la revendication 1 ou 2, **caractérisé en ce que** la microstructure tridimensionnelle (2) possède des zones pouvant être engagées par l'arrière pour la croissance de cellules.

4. Implant selon l'une quelconque des revendications précédentes, **caractérisé en ce que** la structure plane flexible textile (1) est formée par un matériau structurel flexible poreux, notamment un support flexible, et la microstructure tridimensionnelle est formée par la structure de surface exposée (2) du matériau structurel.

5. Implant selon la revendication 4, **caractérisé en ce que** le matériau structurel flexible (2) est un support textile poreux.

6. Implant selon la revendication 5, **caractérisé en ce que** le matériau textile flexible (1), notamment le support textile, présente au moins sur le côté ayant la microstructure tridimensionnelle une structure textile ouverte (2) connue dans le domaine des implants vasculaires, qui est formée notamment par des fils texturés, des flottages et/ou des boucles de velours.

7. Implant selon l'une quelconque des revendications précédentes, **caractérisé en ce que** la structure plane flexible (1), notamment le support textile, est un article tissé ou un article maillée notamment tricoté, qui présente au moins du côté structuré sous forme tridimensionnelle (3) des fibres ou des fils (2) exposés servant d'ancrage pour des cellules.

8. Implant selon l'une quelconque des revendications précédentes, **caractérisé en ce que** la structure plane flexible (1), notamment le support texturé, est un velours (2), notamment un double velours, un double velours présentant sur le côté structuré de préférence une plus grande hauteur de poil que sur le côté essentiellement fermé de l'implant.

9. Implant selon l'une quelconque des revendications précédentes, **caractérisé en ce que** le revêtement (5) est un toison giclé constitué d'un polymère soluble ou dispersable dans un milieu fluide non aqueux, de préférence volatil.

10. Implant selon l'une quelconque des revendications précédentes, **caractérisé en ce que** la couche de surface (5) essentiellement fermée se compose de polyuréthane, notamment de polyuréthane non réticulé.

11. Implant selon l'une quelconque des revendications précédentes, **caractérisé en ce que** l'ensemble de l'implant présente une perméabilité à l'air de 5 à 100 ml d'air/cm² min., pour une différence de pression de 1,2 kPascal.

12. Implant selon l'une quelconque des revendications précédentes, **caractérisé en ce que** la surface (5) essentiellement fermée et notamment l'ensemble de l'implant possède une perméabilité à l'air de 25 à 75 ml d'air/cm² min., pour une différence de pression de 1,2 kPascal.

13. Implant selon l'une quelconque des revendications précédentes, **caractérisé en ce qu'**il présente des bords résistant à l'effilochage, qui sont traités et/ou liés de manière résistant à l'effilochage, notamment qui sont soudés.

14. Implant selon l'une quelconque des revendications 1 à 9 et 11 à 13, **caractérisé en ce qu'**il est partiellement résorbable sur toute la surface, notamment **en ce que** la surface essentiellement fermée se compose d'un matériau complètement résorbable.
